# EUROPEAN PATENT APPLICATION

(11) **EP 3 943 118 A1**
(43) Date of publication of application: **26.01.2022**
(21) Application number: 20773289.2
(22) Date of filing: 11.03.2020
(51) Int. Cl.: A61L 2/10

(54) **DECONTAMINATION METHOD**

(30) Priority: 18.03.2019 JP 2019050430
(71) Applicant: Ushio Denki Kabushiki Kaisha, Tokyo 100-8150 (JP); Director General Of National Institute Of Health Sciences, Kawasaki-shi, Kanagawa 210-9501 (JP)
(72) Inventor: MATSUMOTO,Hiroko, Tokyo 100-8150 (JP); YAMANAKA,Makoto, Tokyo 100-8150 (JP); TAKEMOTO,Fumitoshi, Tokyo 100-8150 (JP); HATAKEYAMA,Kenji, Tokyo 100-8150 (JP); HAISHIMA,Yuji, Kawasaki-shi, Kanagawa 210-9501 (JP); KUDO,Yukiko, Kawasaki-shi, Kanagawa 210-9501 (JP); KIKUCHI,Yutaka, Kawasaki-shi, Kanagawa 210-9501 (JP); FUKUI,Chie, Kawasaki-shi, Kanagawa 210-9501 (JP)
(74) Representative: Tomerius, Isabel
(86) International application number: PCT/JP2020/010501
(87) International publication number: WO 2020/189437

(57) **Abstract**

Disclosed herein is a method for decontaminating an object to be treated by reducing the activity of endotoxin in a simple manner. The decontamination method includes a step (a) of irradiating an object to be treated with ultraviolet light of a wavelength of less than 200 nm to reduce activity of endotoxin attached to the object to be treated.

## Description

### TECHNICAL FIELD

The present invention relates to a decontamination method, and in particular to a method for reducing the activity of endotoxin attached to an object to be treated.

### BACKGROUND ART

Endotoxin entering the bloodstream causes various biological reactions such as fever even when the amount thereof is very small. Therefore, it is necessary to reduce the activity of endotoxin attached to a medical device, especially one implanted in the body.

Examples of a conventionally-known method for reducing the activity of endotoxin include a dry heat treatment method, a method in which an object to be treated is irradiated with radioactive rays such as gamma rays, a method in which an object to be treated is treated by a combination of hydrogen peroxide and ozone, and a method in which an object to be treated is treated using plasma.

Patent Document 1 mentioned below discloses a method for inactivating endotoxin, in which fine streamer discharge is generated by applying electric pulses to an electrode pair in a nitrogen ambient gas to allow a combination of pulse electric field, nitrogen radicals, and ultraviolet light with a wavelength of about 250 nm generated by the nitrogen ambient gas due to fine streamer discharge to act on endotoxin.

Patent Document 2 mentioned below discloses a method of performing sterilization and endotoxin inactivation treatment simultaneously by introducing hydrogen peroxide vapor and ozone gas into a chamber in which the object to be treated is placed, causing hydroxyl radicals to act on the object to be treated.

### PRIOR ART DOCUMENT

### PATENT DOCUMENTS

Patent Document 1: JP-A-2008-178679
Patent Document 2: JP-A-2016-154835

### NON-PATENT DOCUMENT

Non-Patent Document 1: Tsuchiya, "Taking of LAL, Story 2 Endotoxins", Wako News No. 2, 1990, October

### SUMMARY OF THE INVENTION

### PROBLEMS TO BE SOLVED BY THE INVENTION

In the conventional dry-heat treatment method, the treatment temperature is set to about 250°C. This is because endotoxin is a heat-resistant toxin. For example, Non-Patent Document 1 also states that dry-heat sterilization should be performed at a temperature of 250°C or higher for at least 30 minutes to sufficiently inactivate endotoxin.

On the other hand, for example, the glass transition temperature of a cycloolefin polymer (COP) resin is about 140°C and the glass transition temperature of a polycarbonate (PC) resin is about 150°C. That is, the dry-heat treatment method described above can be used for inactivation of endotoxin on an object to be treated made of an inorganic material such as a metal or ceramic, but is difficult to use for inactivation of endotoxin on an object to be treated made of a resin material.

In the case of the method that irradiates an object to be treated with gamma rays or other radioactive rays, it is necessary to provide a shield for blocking the gamma rays during treatment, which increases the scale of an apparatus and requires careful handling.

In the case of the method disclosed in Patent Document 1, it is necessary to create a vacuum environment to generate plasma, which requires various devices such as a pump, a thermostat bath, and a gas removal device, and therefore there is a problem that the scale of an apparatus increases.

In the case of the method disclosed in Patent Document 2, a hydrogen peroxide vapor generator, an ozone gas generator, and a decompressor are required, and therefore an apparatus is expected to increase in size.

Further, when bacterial cells are attached to an object to be treated instead of endotoxin itself, a method for readily inactivating endotoxin constituting the bacterial cells in a short time has not yet been practically used.

Under the circumstances, it is an object of the present invention to provide a method for decontaminating an object to be treated by reducing the activity of endotoxin in a simple manner.

### MEANS FOR SOLVING THE PROBLEMS

The present invention is directed to a decontamination method including a step (a) of irradiating an object to be treated with ultraviolet light of a wavelength of less than 200 nm to reduce activity of endotoxin attached to the object to be treated.

As a result of intensive studies by the present inventors, it has been confirmed that irradiation with ultraviolet light with a wavelength of less than 200 nm has the effect of reducing the activity of endotoxin. Particularly, as will be described later in the section "MODE FOR CARRYING OUT THE INVENTION", it has been confirmed that not only irradiation of endotoxin itself with ultraviolet light but also irradiation of bacterial cells containing endotoxin with ultraviolet light has the effect of reducing the activity of endotoxin. That is, the object to be treated may have a surface on which either the endotoxin itself or bacterial cells containing the endotoxin is attached.

Namely, according to the above method, the activity of endotoxin can be reduced in a simple manner by preparing a light source device that emits ultraviolet light with a wavelength of less than 200 nm and irradiating the object to be treated with the ultraviolet light emitted from the light source device.

Further, as will be described later in the section "MODE FOR CARRYING OUT THE INVENTION", it has been confirmed that the method is effective also in a room temperature environment. Thus, unlike the conventional dry heat treatment method, it is not necessary to place an object to be treated under a high temperature of about 250°C. Therefore, the activity of endotoxin can be reduced even when the object to be treated is made of a resin or the like. For example, in experimental and research institutes, microplates, Petri dishes, preparations, etc. are utilized for cell culture. Recently, synthetic resins have been used as materials thereof. The method according to the present invention can be also applied to reduce the activity of endotoxin on such tools.

The step (a) may be performed in an air atmosphere. As a result of the inventors' intensive studies, it has been confirmed that irradiating an object to be treated with ultraviolet light in an air atmosphere is also effective in reducing the activity of endotoxin attached to the object to be treated. That is, according to the method, it is only necessary to irradiate an object to be treated placed in, for example, a room with ultraviolet light, that is, it is not necessary to perform a step in which a specific atmosphere environment such as a nitrogen atmosphere environment is created in a predetermined closed space, an object to be treated is sealed in the closed space, and the object to be treated is taken out of the closed space after treatment.

That is, the method is useful for manufacturing a tool whose activity of endotoxin is highly reduced. For example, a process of reducing the activity of endotoxin can easily be incorporated as one process of a manufacturing line in a manufacturing plant by conveying tools as objects to be manufactured with a carrier apparatus such as a belt conveyor and sequentially irradiating the tools with ultraviolet light with a wavelength of less than 200 nm emitted from a light source device.

Further, according to the method, it is not necessary to place an object to be treated under a high temperature of, for example, 250°C, and therefore the activity of endotoxin can be reduced even when the object to be treated is made of a resin. For example, the object to be treated may be made of at least one resin material selected from the group consisting of cycloolefin polymer (COP), polycarbonate (PC), polystyrene (PS), silicone, ABS, polyamide (PA), polyvinyl chloride (PVC), and polymethyl methacrylate (PMMA).

The step (a) may be a step of irradiating the ultraviolet light under the temperature above room temperature and below a glass transition temperature of the resin material. As a result of intensive studies by the present inventors, it has been confirmed that when irradiation with the ultraviolet light is performed at a temperature of, for example, 130°C, the effect of reducing the activity of endotoxin is further enhanced as compared to when irradiation with the ultraviolet light is performed at room temperature (20°C). As described above, the glass transition temperature of a cycloolefin polymer (COP) resin is about 140°C and the glass transition temperature of a polycarbonate (PC) resin is about 150°C. Therefore, in a case where an object to be treated is made of such a resin material, a glasstransition phenomenon does not occur even when the object to be treated is placed at a temperature of 130°C. That is, according to the method, it is possible to further enhance the effect of reducing the activity of endotoxin without affecting the material properties of the object to be treated.

The step (a) may be a step of irradiating the object to be treated with the ultraviolet light from an excimer lamp including a tubular body enclosed with a discharge gas containing Xe. At this time, the object to be treated is irradiated with ultraviolet light having a main emission wavelength of 160 nm to 180 nm, more specifically about 172 nm.

The step (a) may be a step of inactivating the endotoxin. In this description, the "inactivating" means that the residual activity ratio of endotoxin is reduced to 0.1% or less (3 Log or less). As will be described later in the section "MODE FOR CARRYING OUT THE INVENTION", it has been confirmed that endotoxin can be inactivated by irradiation with ultraviolet light with a wavelength of less than 200 nm.

### EFFECT OF THE INVENTION

According to the present invention, it is possible to realize a method for decontaminating an object to be treated by reducing the activity of endotoxin in a simple manner.

### BRIEF DESCRIPTION OF THE DRAWINGS

Fig. 1 is a diagram schematically showing an embodiment of a decontamination method according to the present invention.
Fig. 2 is a diagram schematically showing an example of the structure of an excimer lamp.
Fig. 3 is a diagram showing the spectrum of ultraviolet light emitted from an excimer lamp enclosed with a discharge gas containing Xe.
Fig. 4A is a graph showing a change in the residual activity ratio of endotoxin when an object to be treated onto which endotoxin has been applied is irradiated with ultraviolet light emitted from a light source unit #1.
Fig. 4B is a graph showing a change in the residual activity ratio of endotoxin when an object to be treated onto which endotoxin has been applied is irradiated with ultraviolet light emitted from a light source unit #2.
Fig. 4C is a graph showing a change in the residual activity ratio of endotoxin when an object to be treated onto which endotoxin has been applied is irradiated with ultraviolet light emitted from a light source unit #3.
Fig. 4D is a graph showing a change in the residual activity ratio of endotoxin when an object to be treated onto which endotoxin has been applied is irradiated with ultraviolet light emitted from a light source unit #4.
Fig. 4E is a graph showing a change in the residual activity ratio of endotoxin when an object to be treated onto which endotoxin has been applied is irradiated with ultraviolet light emitted from a light source unit #5.
Fig. 4F is a graph showing a change in the residual activity ratio of endotoxin when an object to be treated onto which endotoxin has been applied is irradiated with ultraviolet light emitted from a light source unit #6.
Fig. 5 is a graph showing a change in the residual activity ratio of endotoxin when objects to be treated onto which endotoxin has been applied were irradiated with ultraviolet light emitted from the light source unit #1 under different temperature conditions.
Fig. 6A is a graph showing a change in the residual activity ratio of endotoxin when objects to be treated onto which endotoxin has been applied were irradiated with ultraviolet light emitted from the light source unit #1 in different ambient gases.
Fig. 6B is a partially enlarged diagram of Fig. 6A.
Fig. 7 is a graph showing a change in the residual activity ratio of endotoxin when objects to be treated onto which killed bacterial cells have been applied is irradiated with ultraviolet light emitted from the light source unit #1.
Fig. 8 is a diagram schematically showing another example of the structure of the excimer lamp.
Fig. 9 is a diagram schematically showing still another example of the structure of the excimer lamp.

### MODE FOR CARRYING OUT THE INVENTION

A decontamination method according to the present invention will be described below with reference to the drawings.

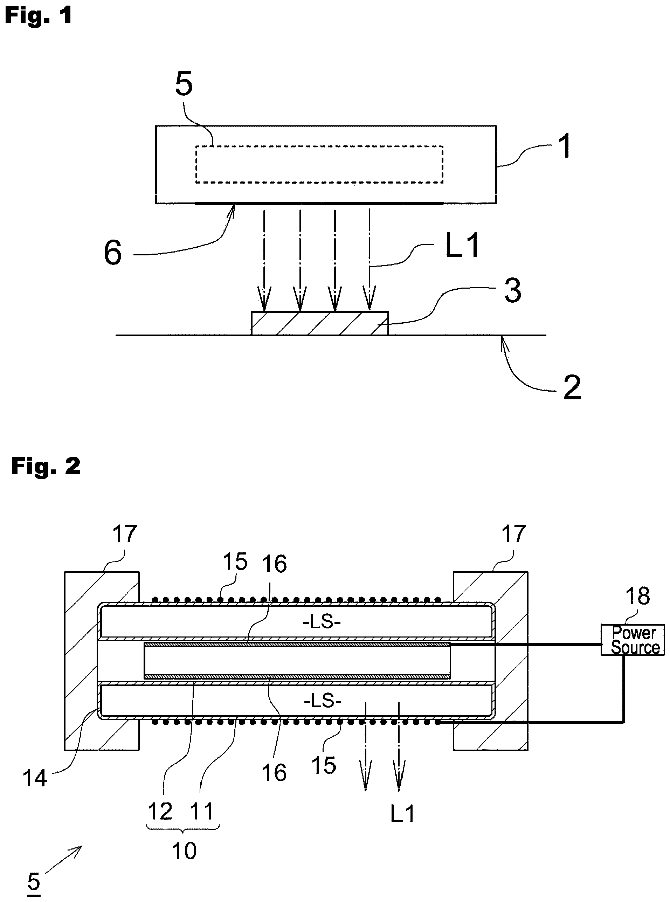

Fig. 1 is a diagram schematically showing an embodiment of the decontamination method according to the present invention. Fig. 1 schematically shows a method for decontaminating an object to be treated 3 by reducing the activity of endotoxin attached to the object to be treated 3. In the example shown in Fig. 1, the decontamination method is carried out by irradiating the object to be treated 3 placed on a predetermined mounting surface 2 with ultraviolet light L1 emitted from a light source device 1 including an excimer lamp 5. The light source device 1 includes a light irradiation window 6 for irradiating the object to be treated 3 with the ultraviolet light L1. The light irradiation window 6 may be constituted from a member that transmits the ultraviolet light L1 (e.g., quartz glass) or may simply be an opening.

Fig. 2 is a cross-sectional view schematically showing an example of the structure of the excimer lamp 5. The excimer lamp 5 includes a light-emitting tube 10 made of a material having the ability to transmit ultraviolet light (e.g., synthetic quartz glass). The light-emitting tube 10 has a cylindrical outer tube 11 and a cylindrical inner tube 12 that is located on the inside of the outer tube 11 coaxially with the outer tube 11 and that has an outer diameter smaller than the inner diameter of the outer tube 11. The outer tube 11 and the inner tube 12 are joined together by sealing walls 14 at both ends in their tube axis directions. This allows an annular light-emitting space LS to be formed between the outer tube 11 and the inner tube 12.

The light-emitting space LS is enclosed with a discharge gas for forming excimer molecules. An example of the discharge gas is a gas containing xenon (Xe). A specific example of the discharge gas is xenon (Xe) alone or a mixed gas containing xenon (Xe) and neon (Ne) in a predetermined ratio (e.g., 3:7). The discharge gas may contain a minute amount of oxygen or hydrogen in addition to xenon (Xe) and neon (Ne).

The excimer lamp 5 includes a mesh-shaped or linear first electrode 15 made of an electrically conductive material such as stainless steel and a film-shaped second electrode 16 made of an electrically conductive material such as aluminum. The first electrode 15 is in close contact with the outer surface of the outer tube 11, and the second electrode 16 is in close contact with the inner surface of the inner tube 12. That is, the first electrode 15 and the second electrode 16 face each other across the light-emitting space LS. In the structure shown in Fig. 2, the first electrode 15 constitutes an outer electrode, and the second electrode 16 constitutes an inner electrode.

The excimer lamp 5 includes bases 17 formed at the ends of the light-emitting tube 10 in the extending direction to surround the outer surface of the light-emitting tube 10. Each of the bases 17 has a closed-bottom tubular shape and is fixed to the light-emitting tube 10 by bringing the inner surface thereof into contact with the outer surface of the outer tube 11 with, for example, an adhesive being interposed therebetween. The bases 17 are made of, for example, ceramic.

The first electrode 15 and the second electrode 16 are both electrically connected to a power source 18. For example, the electrodes (15, 16) and the power source 18 are electrically connected by running a power-supply wire through holes formed in the bottom surface of the base 17. When a high-frequency AC voltage from the power source 18 is applied between the first electrode 15 and the second electrode 16 in the excimer lamp 5, electric discharge occurs in the light-emitting space LS to generate excimer light which depends on the type of discharge gas. As described above, when xenon (Xe) is used as a discharge gas, ultraviolet light L1 having a main wavelength of 172 nm is generated. The ultraviolet light L1 is emitted through the outer surface of the outer tube 11 directly or after reflection by the second electrode 16. The object to be treated 3 placed on the outside of the light source device 1 is irradiated with the ultraviolet light L1.

It is to be noted that as shown in Fig. 1, when the light source device 1 is configured so that the ultraviolet light L1 is extracted from the side where the light irradiation window 6 is provided, a reflective member (not shown) may be provided to reflect the ultraviolet light L1 emitted from the excimer lamp 5 and traveling to the opposite side of the light irradiation window 6 to the light irradiation window 6 side, that is, to the object to be treated 3 side.

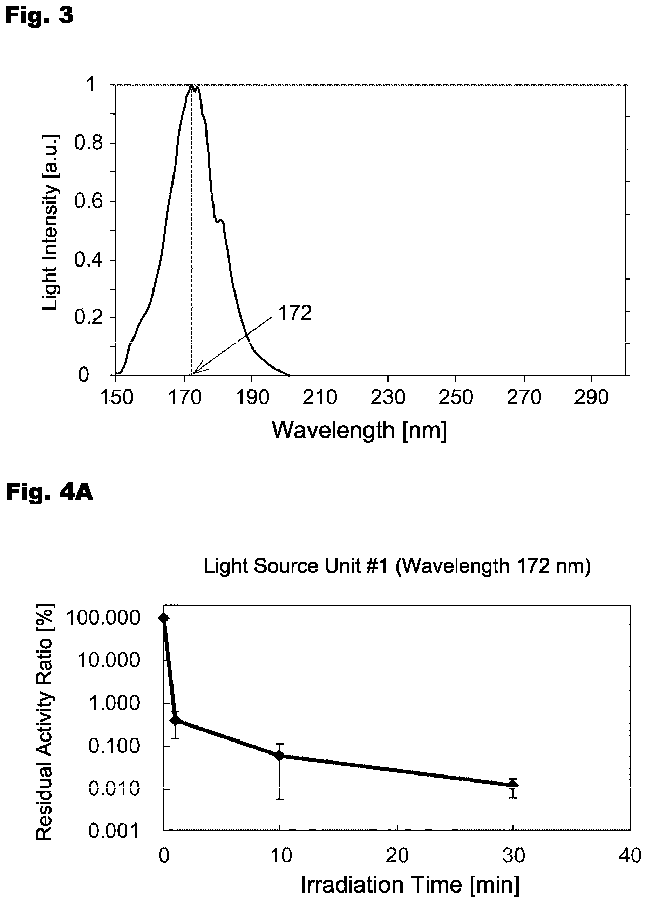

Fig. 3 is a diagram showing the spectrum of ultraviolet light L1 emitted from the light source device 1 when the discharge gas enclosed in the light-emitting space LS contains Xe. The ultraviolet light L1 shows a spectrum having a main emission wavelength of less than 200 nm. More specifically, the ultraviolet light L1 shows a spectrum having a main emission wavelength of 172 nm. However, the spectrum can appropriately be changed by changing the type of discharge gas or by providing a phosphor in an optical path from the light-emitting space LS to the light irradiation window 6. However, in the present invention, the ultraviolet light L1 with which the object to be treated 3 is irradiated has a main emission wavelength of less than 200 nm and preferably has a main emission wavelength of 160 nm to 180 nm.

According to such a method, it is possible to highly reduce the activity of endotoxin attached to the object to be treated 3. Hereinbelow, the present invention will be described with reference to Examples.

In this description, residual activity ratio refers to the ratio of the amount of active endotoxin remaining after treatment to the amount of active endotoxin before treatment.

### EXAMPLES

### (Examination 1)

The relationship between the wavelength of ultraviolet light L1 used for irradiation and the degree of reduction in the activity of endotoxin was examined.

A light source device 1 emitting ultraviolet light L1 and an object to be treated 3 were prepared. As the light source device 1, as will be described later, 6 light source units #1 to #6 were used which were different in the wavelength of ultraviolet light L1 emitted therefrom. The different light source units #1 to #6 were used to change the wavelength of ultraviolet light L1 emitted from the light source device 1. In this way, the degree of reduction in the activity of endotoxin contained in the object to be treated 3 was examined and evaluated. Specifically, the examination and evaluation were performed according to the following procedure.

### (Step S1)

100 EU of endotoxin (LPS: lipopolysaccharide) was applied alone onto a glass test piece (8 mm × 10 mm × 1 mm) subjected to dry-heat sterilization at 250°C for 2 hours, and was then dried for 3 hours. It is to be noted that the "EU" refers to a unit of endotoxin activity defined by the FDA in the US. The unit "EU" is equivalent to "IU" (endotoxin international unit) defined by WHO.

It is to be noted that the dry-heat sterilization at 250°C for 2 hours was performed to prevent a measurement error caused by the initial attachment of endotoxin to the glass test piece. The reason why not a resin piece but a glass test piece is used is that an endotoxin inactivation method now practically used is a dry heat treatment method, and glass is a material that does not change the material properties of a test piece even under a high temperature of 250°C.

### (Step S2)

The glass test pieces obtained in Step S1 were used as objects to be treated 3, and as described in Table 1 shown below, the objects to be treated 3 were irradiated with ultraviolet light L1 different in wavelength emitted from the light source units (#1 to #6). It is to be noted that Step S2 was performed under conditions of an air atmosphere and room temperature.

**[Table 1]**

| Unit name | Wavelength | Light source device | Irradiance on surface of object to be treated 3 [mW/cm²] (Irradiation distance 3 mm) |
|---|---|---|---|
| #1 | 172 nm | Xe excimer lamp | 14 |
| #2 | 190 nm | Xe excimer lamp + Phosphor | 12 |
| #3 | 185 nm & 254 nm | Low-pressure mercury lamp | 2 (185 nm) 18 (254 nm) |
| #4 | 222 nm | KrCI excimer lamp | 7 |
| #5 | 254 nm | Low-pressure mercury lamp | 6 |
| #6 | 320 nm | Xe excimer lamp + Phosphor | 13 |

The details of structures of the light source units #1 to #6 are as follows.

### << Light source unit #1 > >

As the light source unit #1, one including an excimer lamp having an emission peak at about 172 nm (manufactured by Ushio Inc.) was used. Specifically, this excimer lamp had a light-emitting tube enclosed with a discharge gas mainly containing xenon to achieve an emission peak at about 172 nm.

### << Light source unit #2 >>

As the light source unit #2, one including a fluorescent excimer lamp having an emission peak with a wide half-width at about 190 nm (manufactured by Ushio Inc.) was used. Specifically, the fluorescent excimer lamp utilized is one that emits ultraviolet light within a specific wavelength range obtained by excitation of a phosphor irradiated with, as excitation light, light emitted from excimers generated by dielectric barrier discharge. In order to achieve an emission peak with a wide half-width at about 190 nm, this fluorescent excimer lamp had a light-emitting tube filled with a discharge gas mainly containing xenon and had a phosphor made of Y_{0.98}Nd_{0.02} PO₄ having yttrium phosphate as a crystal matrix and activated by trivalent Nd.

### << Light source unit #3 >>

As the light source unit #3, one including a low-pressure mercury lamp emitting ultraviolet light having emission peaks at about 185 nm and about 254 nm was used.

### << Light source unit #4> >

As the light source unit #4, one including a krypton-chloride excimer lamp (manufactured by Ushio Inc.) was used. Specifically, this excimer lamp had a light-emitting tube enclosed with a discharge gas mainly containing krypton and chlorine to achieve an emission peak at about 222 nm.

### << Light source unit #5 >>

As the light source unit #5, one including a low-pressure mercury lamp emitting ultraviolet light having an emission peak at about 254 nm was used. It is to be noted that the low-pressure mercury lamp included in the light source unit #5 had a light-emitting tube made of a glass material not transmitting ultraviolet light of 185 nm.

### << Light source unit #6>>

As the light source unit #6, one including a fluorescent excimer lamp having an emission peak with a wide half-width at about 320 nm (manufactured by Ushio Inc.) was used. The light source unit #6 is different from the light source unit #2 only in the material of the phosphor. Specifically, in order to achieve an emission peak with a wide half-width at 320 nm, this fluorescent excimer lamp had a phosphor made of La_{0.75}Ce_{0.25}PO₄ having lanthanum phosphate as a crystal matrix and activated by trivalent Ce.

In Step S2, the object to be treated 3 was irradiated with ultraviolet light L1 under irradiation conditions shown in Table 1 using each of the light source units #1 to #6.

### (Step S3)

The glass test piece (object to be treated 3) after Step S2 was immersed in endotoxin-free water and subjected to ultrasonic cleaning for 10 to 30 minutes while cooled with ice. An extract after cleaning was mixed with a lysate reagent (Endospecy ES50M manufactured by SEIKAGAKU CORPORATION), and the amount of active endotoxin was measured by a kinetic colorimetric method.

It is to be noted that Step S3 is performed by a method recommended by the National Institute of Health Sciences and compliant with a method recommended in Japanese Pharmacopoeia 17th Edition.

Figs. 4A to 4F are graphs showing the relationship between irradiation time and the residual activity ratio of the object to be treated 3 examined using the light source units #1 to #6 as the light source emitting ultraviolet light L1 in Step S2, respectively. It is to be noted that the vertical axis has a logarithmic scale, and the residual activity ratio [%] represented on the vertical axis is a relative value of the concentration of active endotoxin when the initial concentration of active endotoxin is defined as 100%. It is to be noted that from the viewpoint of confirming the reproducibility of the examination, each of the values obtained as a result corresponds to an average of values obtained by performing Step S3 on three samples subjected to Step S1 and Step S2 under the same conditions.

From Fig. 4A, it is confirmed that when the light source unit #1 emitting ultraviolet light L1 having a main emission wavelength of 172 nm is used in Step S2, the residual activity ratio of endotoxin is reduced to 1% or less by irradiation for 1 minute. Further, it is confirmed that when the irradiation time is 10 minutes, the residual activity ratio of endotoxin is reduced to 0.1% or less as compared to the initial level, that is, 3 Log or less reduction from the initial level can be achieved. From this, it was confirmed that the endotoxin contained in the object to be treated 3 could be inactivated by the light source unit #1. Furthermore, it was confirmed that when the irradiation time was 30 minutes, the residual activity ratio of endotoxin was reduced to about 0.01% as compared to the initial level.

It was confirmed that when the light source unit #2, #3, or #4 was used in Step S2, 30-minute irradiation had the effect of reducing the residual activity ratio of endotoxin as compared to the initial level. However, the degree of the reduction was confirmed to be much lower than thatwhen the light source unit #1 was used.

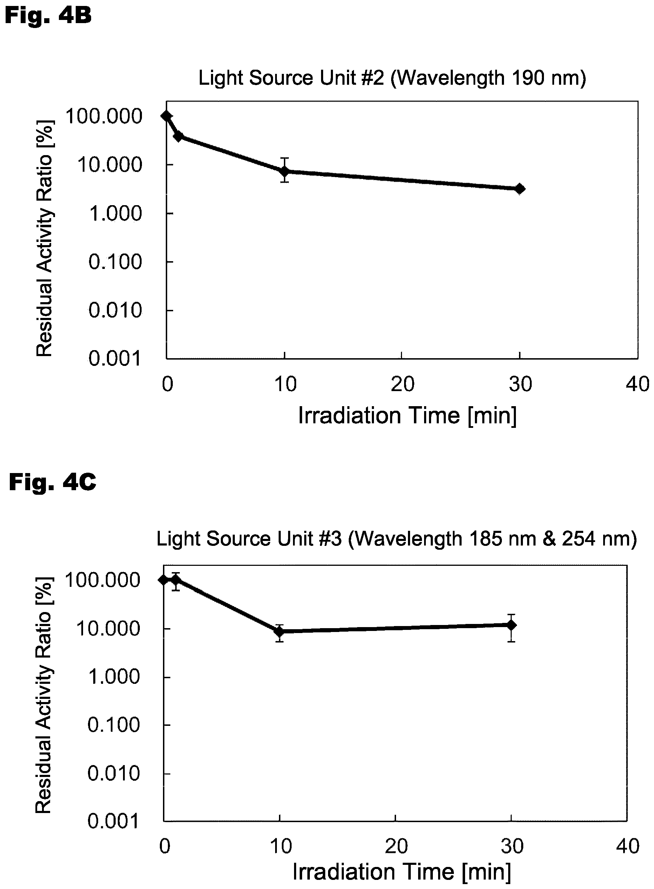

According to Fig. 4B, when the light source unit #2 was used, the residual activity ratio of endotoxin was reduced to about 8% by 10-minute ultraviolet irradiation. Further, the residual activity ratio of endotoxin was reduced to about 3% by 30-minute ultraviolet irradiation. Comparing the reduction tendency of the residual activity ratio of endotoxin when the irradiation time is 10 minutes or less with the reduction tendency of the residual activity ratio of endotoxin when the irradiation time is 10 minutes to 30 minutes, the former is confirmed to be higher. This result reveals that even when the irradiation time is longer than 30 minutes, it is difficult to expect a significant reduction in the residual activity ratio of endotoxin.

According to Fig. 4C, when the light source unit #3 was used, the residual activity ratio of endotoxin was reduced to about 10% by 10-minute ultraviolet irradiation as in the case of using the light source unit #2. However, it was confirmed that the residual activity ratio of endotoxin was increased by 30-minute ultraviolet irradiation as compared to when the amount of irradiation was 10 mJ/cm². The reason for this is not clear, but this result reveals that even when at least the irradiation time is longer than 30 minutes, it is difficult to expect a significant reduction in the residual activity ratio of endotoxin.

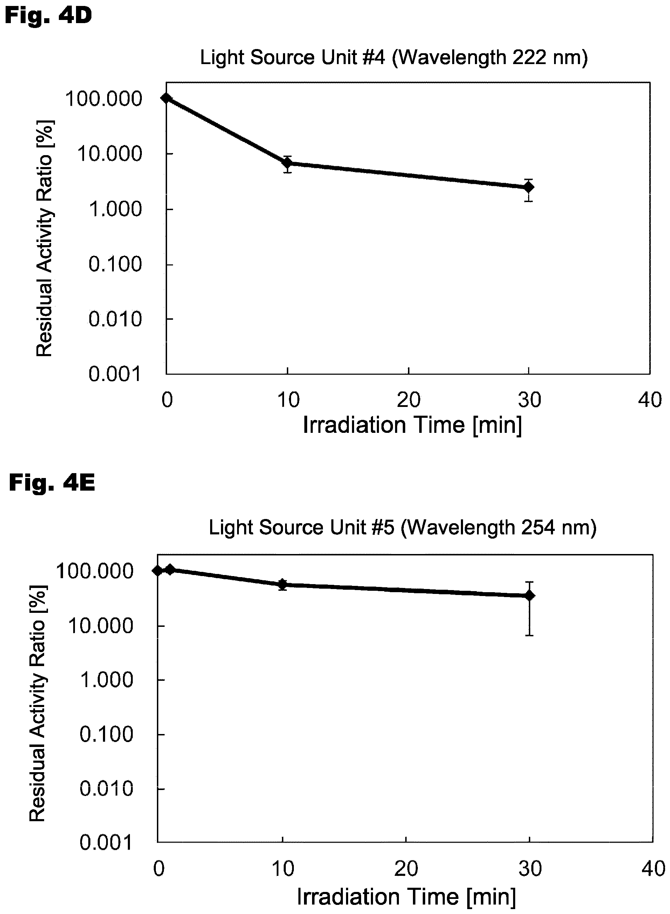

According to Fig. 4D, when the light source unit #4 was used, the residual activity ratio of endotoxin was reduced to about 7% by 10-minute ultraviolet irradiation. Further, the residual activity ratio of endotoxin was reduced to about 2% by 30-minute ultraviolet irradiation. Comparing the reduction tendency of the residual activity ratio of endotoxin when the irradiation time is 10 minutes or less and the reduction tendency of the residual activity ratio of endotoxin when the irradiation time is 10 minutes to 30 minutes, the former is confirmed to be higher. This result reveals that even when the irradiation time is longer than 30 minutes, it is difficult to expect a significant reduction in the residual activity ratio of endotoxin.

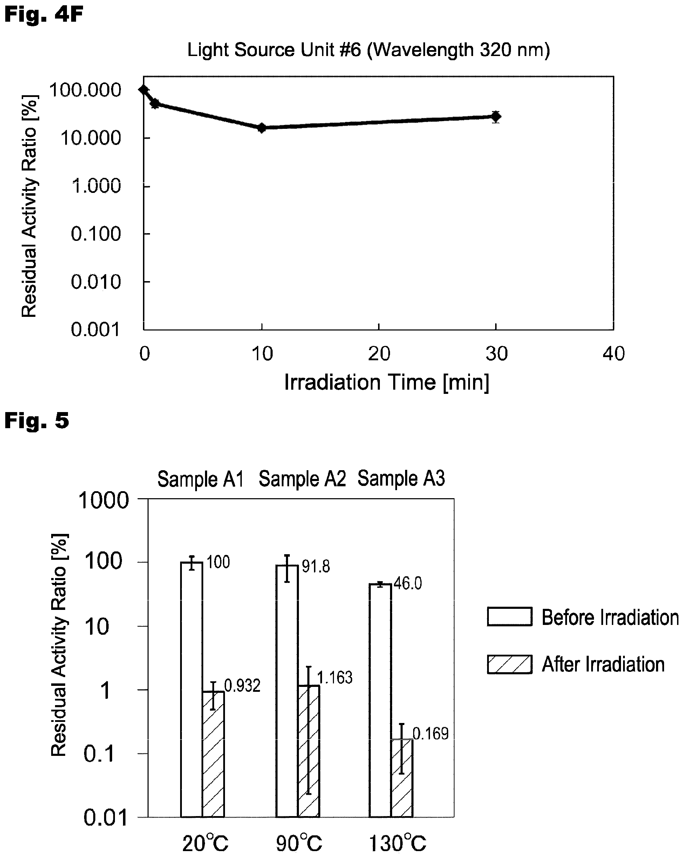

On the other hand, according to Fig. 4E or Fig. 4F, itwas confirmed thatwhen the light source unit #5 or the light source unit #6 was used, the residual activity ratio of endotoxin was hardly changed irrespective of the amount of irradiation.

From the above results, it is confirmed that the residual activity ratio of endotoxin can be reduced, that is, the amount of active endotoxin can be reduced by irradiating the object to be treated 3, to which endotoxin is attached, with ultraviolet light L1 having a main emission wavelength of less than 200 nm. Further, it is confirmed that the residual activity ratio of endotoxin can be reduced to 0.1% or less and inactivated by irradiating the object to be treated 3, to which endotoxin is attached, with ultraviolet light L1 having a main emission wavelength of 172 nm for 10 minutes or more.

### (Examination 2)

The relationship between the ambient temperature during ultraviolet light L1 irradiation and the degree of reduction in the activity of endotoxin was examined. It is to be noted that in all Examination 2 and Examinations 3 to 5 described later, ultraviolet light L1 having a main emission wavelength of 172 nm was used.

### < < Sample A1 > >

A sample obtained by performing Steps S1 to S2 described above was used as a sample A1. It is to be noted that in Step S2, irradiation with ultraviolet light L1 was performed using the light source unit #1 (wavelength: 172 nm) at room temperature (20°C). At this time, the irradiance of ultraviolet light L1 on the surface of the object to be treated 3 was 14 mW/cm², and the irradiation time was 1 minute.

### << Sample A2>>

A sample obtained by performing Step S1A described below instead of Step S1 and then performing Step S2A described below instead of Step S2 was used as a sample A2.

### (Step S1A)

50 EU of endotoxin (LPS) was applied alone onto a glass test piece (6 mm × 6 mm × 1 mm) subjected to dry-heat sterilization at 250°C for 2 hours and dried overnight.

### (Step S2A)

The glass test piece obtained in Step S1A was placed on a hot plate set at 90°C, and in such a state, the glass test piece was irradiated with ultraviolet light L1 with a wavelength of 172 nm emitted from the light source unit #1 for 1 minute. At this time, the irradiance on the surface of the object to be treated 3, that is, the irradiance on the glass test piece was 14 mW/cm².

### << Sample A3 >>

A sample obtained by performing Step S1A and Step S2A in the same manner as when the sample A2 was prepared except that the set temperature was changed to 130°C was used as a sample A3.

### (Step S3)

The residual activity ratio of endotoxin of each of the samples A1 to A3 was measured in the same manner as in Step S3 performed in Examination 1. The results are shown in Table 2 below and Fig. 5. It is to be noted that for comparison, Step S3 was performed in the same manner on a sample (hereinafter referred to as a "comparative sample") prepared under the same temperature conditions as each of the above samples without irradiation with ultraviolet light L1. In Table 1, data in the column "before ultraviolet irradiation" corresponds to results based on the comparative samples.

**[Table 2]**

| Samples | Temperature [°C] | Residual activity [%] | |
|---|---|---|---|
| | | Before ultraviolet irradiation | After ultraviolet irradiation |
| A1 | 20 | 100 | 0.932 |
| A2 | 90 | 91.8 | 1.163 |
| A3 | 130 | 46.0 | 0.169 |

A comparison between the sample A1 and the sample A2 before ultraviolet light L1 irradiation reveals that the effect of reducing the activity of endotoxin can hardly be obtained simply by heating to 90°C. Further, a comparison between the sample A1 and the sample A3 before ultraviolet light L1 irradiation reveals that the residual activity ratio of endotoxin is reduced to about 46% by heating to 130°C, which is however far from inactivation.

Further, a comparison between the sample A1 and the sample A2 reveals that there is little difference between the effect obtained by ultraviolet light L1 irradiation at 90°C with heating and the effect obtained by ultraviolet light L1 irradiation at room temperature (20°C). On the other hand, a comparison between the sample A1 and the sample A3 reveals that the residual activity ratio is reduced to 0.93% by ultraviolet light L1 irradiation at room temperature, whereas the residual activity ratio is 0.169% when ultraviolet light L1 irradiation is performed at 130°C with heating and the residual activity ratio is further reduced by 0.37% as compared to a residual activity ratio of 46.0% achieved by heating to 130°C without ultraviolet light L1 irradiation. Further, a comparison with a state before ultraviolet light L1 irradiation at room temperature reveals that the residual activity ratio is reduced to 0.17% by ultraviolet light L1 irradiation at 130°C with heating.

This means that the speed of a reduction in the activity of endotoxin is increased by irradiating the object to be treated 3 with ultraviolet light L1 in a state where the object to be treated 3 is heated to 130°C. That is, it is revealed that the resid ual activity ratio of endotoxin attached to the object to be treated 3 can significantly be reduced when the object to be treated 3 is irradiated with ultraviolet light L1 for a short time while being heated to 130°C.

As described above, in the conventional dry heat treatment method, the treatment temperature is set to about 250°C. The reason for this is that it has been believed that heating to about 250°C is essential for obtaining the effect of sufficiently reducing the activity of endotoxin. However, it is confirmed from Examination 2 that the effect of reducing the activity of endotoxin can significantly be enhanced by performing ultraviolet light L1 irradiation and heating in combination even when the heating temperature is as low as 130°C. This method can suitably be used particularly when the object to be treated 3 is made of resin.

### (Examination 3)

The relationship between the treatment atmosphere during ultraviolet light L1 irradiation and the degree of reduction in the activity of endotoxin was examined.

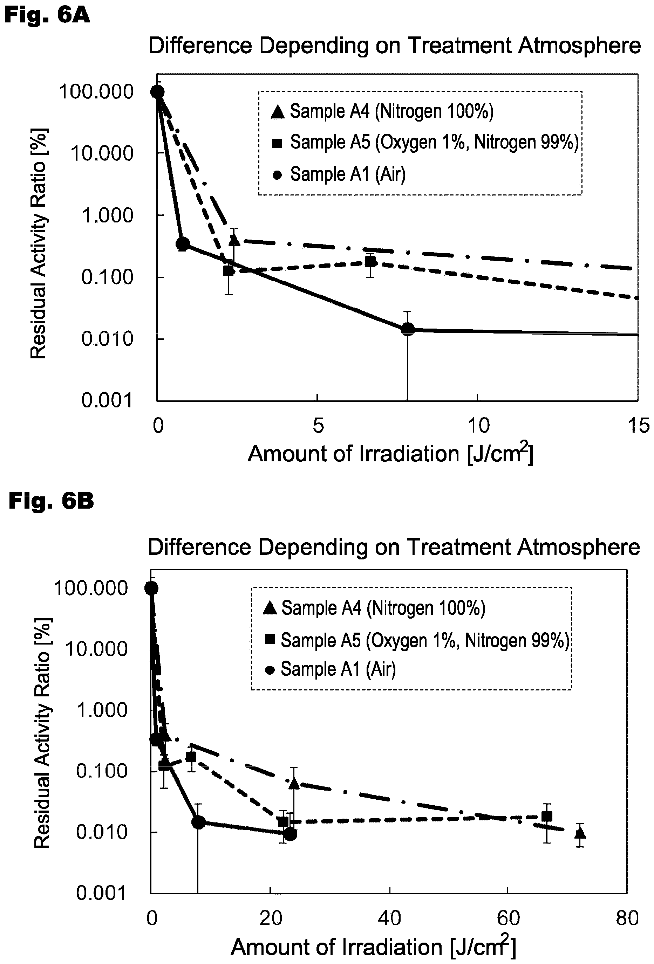

In Examination 1 and Examination 2 described above, Step S2 was performed in an air atmosphere. On the other hand, a sample (sample A4) was prepared by performing Step S1 and then performing Step S2 in a nitrogen atmosphere, a sample (sample A5) was prepared by performing Step S1 and then performing Step S2 in an atmosphere containing 1% oxygen and 99% nitrogen, and the residual activity ratio of endotoxin was measured in the same manner as in Step S3 performed in Examination 1. The results are shown in Fig. 6A and Fig. 6B. Fig. 6A and Fig. 6B are graphs showing the relationship between the amount of ultraviolet light L1 irradiation (product of irradiance and irradiation time) and the residual activity ratio of endotoxin. It is to be noted that Fig. 6B is a graph obtained by expanding the graph in Fig. 6A by changing the range of the horizontal axis to 0 to 15 J/cm².

It is to be noted that in Fig. 6A and Fig. 6B, the result of the sample A1 used in Examination 2 is also shown for comparison. Further, the wavelength of ultraviolet light L1 used for irradiation in Step S2 and the treatment temperature were the same as those for the sample A1 in Examination 2.

As described above, in Examination 3, ultraviolet light L1 having a main emission wavelength of 172 nm was used. This ultraviolet light L1 is highly absorbed by oxygen, and therefore the irradiance on the surface of the sample varies depending on the concentration of oxygen in an atmosphere. For example, the irradiance in an air atmosphere is 14 mW/cm², the irradiance in an atmosphere containing 1% oxygen and 99% nitrogen is about 37 mW/cm², and the irradiance in a nitrogen atmosphere is about 40 mW/cm².

According to Fig. 6B, when a comparison is made among when the treatment atmosphere in which the object to be treated 3 is irradiated with ultraviolet light L1 in Step S2 is nitrogen, when the treatment atmosphere contains 1% oxygen, and when the treatment atmosphere is air, the residual activity ratio of endotoxin decreases in this order. Further, Fig. 6B reveals that in the case of an air atmosphere among these three atmospheres, the residual activity of endotoxin is reduced to less than 0.1%, that is, endotoxin can be inactivated by irradiating endotoxin with ultraviolet light L1 in a minimum amount of irradiation of about 8 J/cm².

This result also suggests that endotoxin can be inactivated by irradiating the object to be treated 3 with a smaller amount of ultraviolet light L1 in an air atmosphere. It is to be noted that Fig. 6A suggests that the residual activity ratio is reduced to less than 0.1%, that is, endotoxin can be inactivated also in an atmosphere whose oxygen concentration is lower than air, such as a nitrogen atmosphere, by increasing the amount of ultraviolet light L1 irradiation of endotoxin.

### (Examination 4)

The degree of reduction in the activity of endotoxin was examined when not endotoxin alone but bacterial cells constituting endotoxin were irradiated with ultraviolet light L1.

In light of availability, the activity of endotoxin alone is conventionally evaluated as an indicator of the inactivation of toxin. However, there are few cases where endotoxin itself is attached to the object to be treated 3, and in most cases, bacterial cells containing endotoxin are attached to the object to be treated 3. That is, from the viewpoint of reducing the activity of endotoxin (or inactivating endotoxin) by treating the object to be treated 3 to which bacterial cells containing endotoxin are attached, it is important to make an evaluation in a state and environment approximating reality, i.e., to which the bacterial cells are attached.

In light of such circumstances, the degree of reduction in the activity of endotoxin was examined by irradiating an object to be treated 3, to which actual bacterial cells are attached, with ultraviolet light L1. A specific procedure is as follows.

### (Step S4)

Dry killed bacterial cells derived from E. coli were applied onto a glass test piece (10 mm × 26 mm × 1 mm or 6 mm × 6 mm × 1 mm) subjected to dry-heating sterilization at 250°C for 2 hours. The amounts of dry killed bacterial cells applied were of four kinds: 1.9 ng/mm ^{2,} 4.8 ng/mm², 14 ng/mm², and 140 ng/mm². Then, the glass test piece was dried overnight. The glass test piece obtained in Step S4 was used as an object to be treated 3. It is to be noted that more specifically when a sample was prepared by applying killed bacterial cells in an amount of 1.9 ng/mm² or 4.8 ng/mm², a glass test piece of 10 mm × 26 mm × 1 mm was used, and when a sample was prepared by applying killed bacterial cells in an amount of 14 ng/mm ² or 140 ng/mm², a glass test piece of 6 mm × 6 mm × 1 mm was used.

### (Step S2)

As in the case of Step S2 in Examination 1, the object to be treated 3 was irradiated with ultraviolet light with a wavelength of 172 nm using the light source unit #1 at room temperature and irradiance of 14 mW/cm² in an air atmosphere.

### (Step S3)

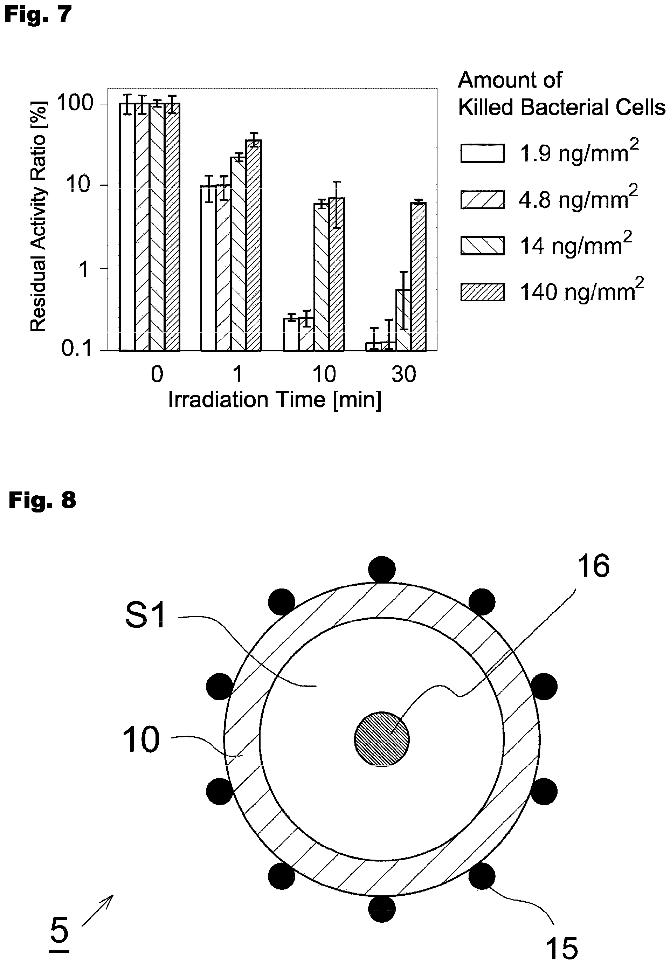

The residual activity ratio of endotoxin contained in the glass test piece (object to be treated 3) after Step S2 was measured in the same manner as in Step S3 in Examination 1. The results are shown in Fig. 7. Fig. 7 is a graph showing the relationship between the ultraviolet light L1 irradiation time and the residual activity ratio of endotoxin.

It was confirmed from the results shown in Fig. 7 that the residual activity ratio of endotoxin could be reduced also by irradiating not endotoxin itself but killed bacterial cells with ultraviolet light L1. From this, it was experimentally confirmed that the activity of endotoxin constituting bacterial cells could be reduced simply by irradiating the object to be treated 3 to which bacterial cells were attached with ultraviolet light L1.

### (Examination 5)

For example, in a case where an object to be treated 3 is made of a resin material, a practical problem may arise when another toxic material is generated by irradiating the object to be treated 3 with ultraviolet light L1. Particularly, medical devices are required to undergo a cytotoxic test for assessment of the toxicity of eluted materials from samples.

Therefore, a cytotoxic test was performed based on a colony formation assay described in "Guidance on Test Methods for Biological Safety Evaluation of Medical Devices (PFSB/ELD/OMDE Notification No. 0301-20)" set by the Ministry of Health, Labour, and Welfare. A specific procedure is as follows.

### (Step S8)

Samples A11 to A18 made of resin materials listed below in Table 3 were prepared and were irradiated with ultraviolet light L1 with a wavelength of 172 nm in the same manner as in Step S2 in Examination 1. The ultraviolet light L1 irradiation time in Step S8 was set to 60 minutes that was twice the maximum irradiation time in Examination 4. This is sufficiently long to perform treatment using ultraviolet light L1.

### (Step S9)

Each of the samples A11 to A18 after Step S8 was cut to a size of about 2 × 15 mm, and then 10 mL of a culture medium was added per 60 cm² of surface area or 1 g to perform extracting for 24 hours. Then, V79 cells (derived from a Chinese hamster) were cultured for 6 days using the extraction medium, and the number of colonies was counted. The colony formation ratio was calculated from the counted number of colonies and based on this value, the value of IC₅₀ (50% inhibition concentration) was calculated. When the colony formation ratio was reduced by more than 30% as compared to a case using an ordinary culture medium, the sample was determined to exert a cytotoxic action.

The results are shown in Table 3. It is to be noted that in Table 3, "ZEONEX" in the type name of a material to be evaluated used in the sample A11, "TPS" in the type name of a material to be evaluated used in the samples A15 and A16, and "COMOGLAS" in the type name of a material to be evaluated used in the sample A17 are all registered trademarks.

**[Table 3]**

| Samples | Material to be evaluated | | | Cytotoxic potency IC₅₀ value (%) | | Change of cytotoxic potency |
|---|---|---|---|---|---|---|
| | Material | Type | Manufacturer | Before irradiation | After irradiation | |
| A11 | Cycloolefin polymer (COP) | ZEONEX6 90R | Zeon Corporation | >100 | >100 | No change |
| A12 | Polycarbonate (PC) | PC-1600 | C.I. TAKIRON Corporation | >100 | >100 | No change |
| A13 | Polystyrene (PS) | PS2031 | HIKARI CO., LTD. | >100 | >100 | No change |
| A14 | Silicone | SR50 | Tigers Polymer Corporation | >100 | >100 | No change |
| A15 | ABS (ABS) | TPS-ABS | Toray Plastics Precision Co., Ltd. | >100 | >100 | No change |
| A16 | Polyamide (PA6) | TPS-N6 | Toray Plastics Precision Co., Ltd. | >100 | >100 | No change |
| A17 | Polymethyl methacrylate (PMMA) | COMOGL AS | Kuraray Co., Ltd. | >100 | 81.5 | Weak cytotoxicity |
| A18 | Polyvinyl chloride (PVC) | ESS8800A | C.I. TAKIRON Corporation | 34.1 | 36.4 | No change |

According to the results shown in Table 3, in the case of the sample A17 made of a polymethyl methacrylate resin, the value of IC₅₀ was confirmed to be reduced by irradiation with ultraviolet light L1, that is, weak cytotoxicity was observed. However, the amount of reduction was less than 30%, and therefore it can be concluded that the sample 17 does not exert a cytotoxic action.

On the other hand, in all cases of the samples A11 to A16, the values of IC₅₀ were not changed. In the case of the sample A18, middle cytotoxicity was observed before irradiation with ultraviolet light L1, but the value of IC₅₀ was not reduced, that is, toxicity was not increased by irradiation with ultraviolet light L1. These results indicate that decontamination performed on various resin materials made of a cycloolefin polymer (COP), a polycarbonate (PC), polystyrene (PS), silicone, ABS, a polyamide (PA), and polyvinyl chloride (PVC) according to the method of the present invention causes no problem in terms of a cytotoxic test.

### [Other Embodiments]

Hereinbelow, other embodiments will be described.

<1> In the present invention, the structure of the light source device 1 used for irradiation with ultraviolet light L1 is not limited to the example shown in Fig. 2. For example, when the light source device 1 includes an excimer lamp, the excimer lamp may have a light-emitting tube 10 having a so-called "single tube structure" or "flattened tube structure" other than a so-called "double tube structure" shown in Fig. 2.

Fig. 8 is a schematic diagram showing the structure of an excimer lamp 5 having a so-called "single tube structure", which is viewed in the longitudinal direction (tube axis direction). Unlike the excimer lamp 5 shown in Fig. 2, the excimer lamp 5 shown in Fig. 8 has a light-emitting tube 10 constituted from one tubular body. The light-emitting tube 10 is sealed at the ends thereof (not shown) in the longitudinal direction, forming a light-emitting space LS inside itself. The space S1 is enclosed with a discharge gas. In the inside of the tubular body of the light-emitting tube 10, a second electrode 16 is provided, and on the outer wall surface of the light-emitting tube 10, a mesh-shaped or linear first electrode 15 is provided.

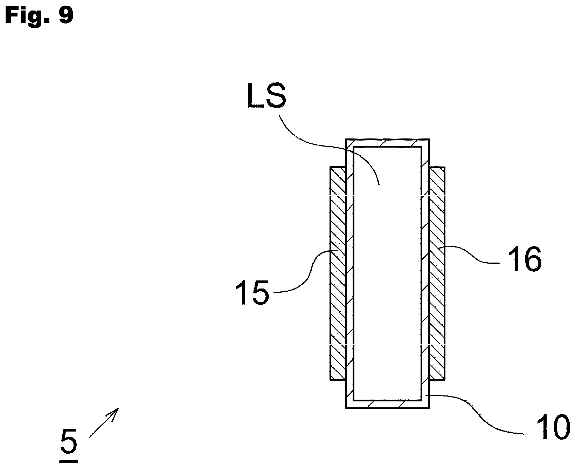

Following Fig. 8, Fig. 9 is a schematic diagram showing the structure of an excimer lamp 5 having a so-called "flattened tube structure". The excimer lamp 5 shown in Fig. 9 has a light-emitting tube 10 constituted from one tubular body having a rectangular shape when viewed in the longitudinal direction. The excimer lamp 5 has a first electrode 15 provided on the outer surface of the light-emitting tube 10 and a second electrode 16 provided on the outer surface of the light-emitting tube 10 to be opposed to the first electrode 15. Both the first electrode 15 and the second electrode 16 have a mesh shape (net shape) or a linear shape so as not to interfere with the emission of ultraviolet light L1 generated in the light-emitting tube 10 to the outside of the light-emitting tube 10.

Fig. 8 shows a case where the excimer lamp 5 has a circular shape when viewed in the longitudinal direction. This goes for the excimer lamp 5 having the structure shown in Fig. 2. Fig. 9 shows a case where the shape is rectangular. However, the shape of the excimer lamp 5 when the excimer lamp 5 is viewed in the longitudinal direction is not limited to a circular or rectangular shape, and various shapes may be employed.

<2> The method according to the present invention can be applied not only to a case where the object to be treated 3 is made of the resin material mentioned above in Examination 5 but also to a case where the object to be treated 3 is made of a glass material, a metallic material, a ceramic material, or the like.

### DESCRIPTION OF REFERENCE SIGNS

- 1: Light source device
- 2: Mounting surface
- 3: Object to be treated
- 5: Excimer lamp
- 6: Light irradiation window
- 10: Light-emitting tube
- 11: Outer tube
- 12: Inner tube
- 14: Sealing wall
- 15: First electrode
- 16: Second electrode
- 17: Base
- 18: Power source
- L1: Ultraviolet light
- LS: Light-emitting space

## Claims

1. A decontamination method comprising a step (a) of irradiating an object to be treated with ultraviolet light of a wavelength of less than 200 nm to reduce activity of endotoxin attached to the object to be treated.

2. The decontamination method according to claim 1, wherein the object to be treated has a surface on which the endotoxin itself or bacterial cells containing the endotoxin is attached.

3. The decontamination method according to claim 1 or 2, wherein the step (a) is performed in an air atmosphere.

4. The decontamination method according to any one of claims 1 to 3, wherein the object to be treated is made of at least one resin material selected from the group consisting of a cycloolefin polymer, a polycarbonate, polystyrene, silicone, ABS, a polyamide, polyvinyl chloride, and polymethyl methacrylate.

5. The decontamination method according to claim 4, wherein the step (a) is a step of irradiating the ultraviolet light under the temperature above room temperature and below a glass transition temperature of the resin material.

6. The decontamination method according to any one of claims 1 to 5, wherein the step (a) is a step of irradiating the object to be treated with the ultraviolet light from an excimer lamp including a tubular body enclosed with a discharge gas containing Xe.

7. The decontamination method according to any one of claims 1 to 6, wherein the step (a) is a step in which the endotoxin is inactivated.
